# EUROPEAN PATENT SPECIFICATION

(11) **EP 2 756 841 B1**
(45) Date of publication and mention of the grant of the patent: **13.05.2015**
(21) Application number: 13152083.5
(22) Date of filing: 21.01.2013
(51) Int. Cl.: A61K 9/16, A61K 31/381

(54) **Pharmaceutical compositions comprising an acid salt**
Pharmazeutische Zusammensetzungen mit einem Säuresalz
Compositions pharmaceutiques comprenant un sel d'acide

(43) Date of publication of application: 23.07.2014
(73) Proprietor: Galenicum Health S.L., 08005 Barcelona (ES)
(72) Inventor: Cubel Suñé, Núria, 08005 Barcelona (ES)
(74) Representative: Galenicum Health S.L.

(56) References cited:
- EP-A1- 2 335 704
- CN-A- 1 839 827
- CN-A- 101 530 617
- Unknown: "Protelos- Scientific Discussion", Protelos, 16 November 2005 (2005-11-16), pages 1-23, XP055059321, Internet Retrieved from the Internet: URL:http://www.ema.europa.eu/docs/en_GB/do cument_library/EPAR_-_Scientific_Discussio n/human/000560/WC500045522.pdf [retrieved on 2013-04-11]
- Unknown: "Protelos Summary of Product Characteristics", Protelos Summary of Product Characteristics., 11 October 2012 (2012-10-11), pages 1-32, XP055059294, Internet Retrieved from the Internet: URL:http://www.ema.europa.eu/docs/en_GB/do cument_library/EPAR_-_Product_Information/ human/000560/WC500045525.pdf [retrieved on 2013-04-11]
- Unknown: "Protelos - Procedural steps taken and scientific information after the authorisation", Protelos, 7 November 2012 (2012-11-07), pages 1-10, XP055059299, Internet Retrieved from the Internet: URL:http://www.ema.europa.eu/docs/en_GB/do cument_library/EPAR_-_Procedural_steps_tak en_and_scientific_information_after_author isation/human/000560/WC500045526.pdf [retrieved on 2013-04-11]

## Description

The present invention relates to pharmaceutical compositions, preferably in the form of granules, which can be used in the treatment of osteoporosis.

Throughout this application various publications, published patent applications, and patents are referenced. The disclosures of these documents in their entirety are hereby incorporated by reference into this application in order to more fully describe the state of the art to which this invention pertains.

### STATE OF THE ART

An appropriate pharmaceutical carrier system is generally a requirement for the safe and effective delivery of a pharmaceutical active ingredient. The entire pharmaceutical composition, i.e. the pharmaceutical active ingredient formulated in a pharmaceutical carrier, can affect the bioavailability and also the pharmacokinetics and pharmacodynamics of the active ingredient. It is therefore important that a pharmaceutical composition be carefully developed and manufactured to deliver the desired pharmaceutical active in a safe and effective manner. Therefore, the development and manufacture of suitable pharmaceutical compositions for the safe and effective delivery of pharmaceutical drug actives are important and ongoing needs.

Convenience of administration and patient compliance are gaining significant importance in the design of dosage forms. Recently, more stress is laid down on the development of an organoleptically elegant and patient-friendly drug delivery system for paediatric and geriatric patients. More than 50 % of the pharmaceutical products are orally administered for several reasons, and undesirable taste is one of the important formulation problems encountered with such oral products. Taste of a pharmaceutical product is an important parameter for governing compliance. Thus, taste masking of oral pharmaceuticals has become an important tool to improve patient compliance and the quality of treatment. Therefore, formulation of taste-masked products is a challenge to the pharmacists. In addition, it is important to develop compositions which can be administered highly concentrated, in order to reduce the amount of the pharmaceutical composition to be taken.

Several pharmaceutical processes, including blending, transfer, storage, feeding, compaction, and fluidization, involve powder handling. (The term powder is used predominantly throughout art, but these concepts also apply to other bulk solids - fine and coarse - such as dust, granulations, and granules either as single substances or as multicomponent blends). The flow of powder during manufacturing dictates the quality of the product in terms of its weight and content uniformity. Flow also affects manufacturing efficiency, because it can determine whether bins can be used or hand scooping will be required, to what extent product (if any) is scrapped at the beginning or end of the run, and the allowable production rate of product (e.g., blend times and compression speeds). However, much proven scientific understanding of bulk powder flow has not been used fully by the pharmaceutical industry. The active ingredients normally have poor flowability, as for example the agent useful for the treatment of osteoporosis known as the distrontium salt of the acid 2-(N,N-di(carboxymethyl)amino)-3-cyano-4-carboxymethyl-5-carboxythiophene.

The marketed product Protelos® comprises the agent and is also mentioned in EP2335704.

J There is a need in the art, in order to improve patient compliance, of pharmaceutical compositions which are patient friendly with a high content of active pharmaceutical ingredient, especially in the cases where the active ingredient has poor flow properties.

### SUMMARY OF THE INVENTION

Therefore, the present invention has been made in view of the above problems, and it is an object of the present invention to provide a stable pharmaceutical composition comprising a high concentration of the active ingredient with good bioavailability and good physical properties, in spite of the high concentration of the active ingredient. Another advantage of the pharmaceutical compositions as herein disclosed is that they can be packaged in stick-packs due to good flowability. The present invention also provides pharmaceutical compositions comprising the distrontium salt of the acid 2-(N,N-di(carboxymethyl)amino)-3-cyano-4-carboxymethyl-5-carboxythiophene and packaged in stick-packs. In addition, the present invention relates to pharmaceutical compositions showing good pharmaceutical properties, as fast onset, a good flowability and being stable over the time. Moreover, the pharmaceutical compositions as herein disclosed can be formulated with high content of active ingredient. The present invention relates also to process to manufacture the composition as herein disclosed, which provides reproducible results.

In the first aspect, it is provided a pharmaceutical composition in the form of immediate release granules, wherein the pharmaceutical composition comprises:
a) an agent from 25 to 85 % w/w in respect of the total amount of the pharmaceutical composition;
b) up to 15.7 % w/w of water content in respect of the total amount of the pharmaceutical composition or the ratio water content / agent measured as anhydrous form is up to 31.5 % w/w;
   wherein the agent is the distrontium salt of the acid 2-(N,N-di(carboxymethyl)amino)-3-cyano-4-carboxymethyl-5-carboxythiophene, and wherein the D50 of the immediate release granules ranges from 140 to 800 microns and, advantageously, the D90 is below 1600 microns.

In the second aspect, it is provided a process for the manufacture of a pharmaceutical composition in the form of immediate release granules, wherein the pharmaceutical composition comprises:
a) an agent from 25 to 85 % w/w in respect of the total amount of the pharmaceutical composition;
b) up to 15.7 % w/w of water content in respect of the total amount of the pharmaceutical composition or the ratio water content / agent measured as anhydrous form is up to 31.5 % w/w;
wherein the agent is the distrontium salt of the acid 2-(N,N-di(carboxymethyl)amino)-3-cyano-4-carboxymethyl-5-carboxythiophene, and, advantageously, the agent is the distrontium salt of the acid 2-(N,N-di(carboxymethyl)amino)-3-cyano-4-carboxymethyl-5-carboxythiophene; and wherein the D50 of the immediate release granules ranges from 140 to 800 microns and, advantageously, the D90 is below 1600 microns; wherein the process for the manufacture of the pharmaceutical composition in the form of immediate release granules comprises the steps of:
i) sieving when appropriate the intra-granular ingredients to form uniformly sized particles of intra-granular ingredients;
ii) mixing the particles of intra-granular ingredients and the distrontium salt of the acid 2-(N,N-di(carboxymethyl)amino)-3-cyano-4-carboxymethyl-5-carboxythiophene, preferably in a granulator-mixer; granulate the obtained mixture with at least one binder, preferably the binder comprises water and a binder agent, more preferably the binder consists essentially of water and maltodextrin;
iii) optionally and/or if required, performing a sieving of the granulate obtained in step (ii);
iv) drying the granulate of the previous step (ii) or (iii) to an endpoint up to 15.7 % w/w of water content in respect of the total amount of the granulate or the ratio water content / agent measured as anhydrous form is up to 31.5 % w/w, wherein the drying is preferably carried out in a fluid bed at an input temperature between 35 and 105 °C.

In the third aspect, it is provided a pharmaceutical batch of at least 10,000 units of the pharmaceutical composition as herein disclosed.

In the fourth aspect, it is provided a pharmaceutical composition comprising the distrontium salt of the acid 2-(N,N-di(carboxymethyl)amino)-3-cyano-4-carboxymethyl-5-carboxythiophene, wherein the pharmaceutical composition is in the form of a stick-pack, wherein the pharmaceutical composition comprises at least the following two portions: i) a granulate; ii) a stick package, wherein the stick package comprises at least a sealed laminated foil of aluminium and, preferably, the granulate comprises an amount of the distrontium salt of the acid 2-(N,N-di(carboxymethyl)amino)-3-cyano-4-carboxymethyl-5-carboxythiophene measured as anhydrous form ranging from 1.75 to 2.25 grams in each stick-pack.

### DETAILED DESCRIPTION OF THE INVENTION

In a further embodiment of the pharmaceutical composition as herein disclosed, the agent is the distrontium salt of the acid 2-(N,N-di(carboxymethyl)amino)-3-cyano-4-carboxymethyl-5-carboxythiophene. Such agent is also known as the distrontium salt of the ran-elic acid, strontium 2,2'-((5-carboxylato-4-(carboxylatomethyl)-3-cyanothiophen-2-yl)azanediyl)diacetate, strontium salt (1:2) of 5-(bis(carboxymethyl)amino)-2-carboxy-4-cyano-3-thiopheneacetic acid, strontium 5-[bis(carboxymethyl)amino]-3-(carboxymethyl)-4-cyano-2-thiophenecarboxylate or strontium 5-[bis(2-hydroxy-2-oxoethyl)amino]-4-cyano-3-(2-hydroxy-2-oxoethyl)thiophene-2-carboxylate (see for example CA2442881, application number CA20032442881). The CN application CN1839827, discloses tablets comprising the agent and the patent application CN101204375 relates to dry suspension comprising the agent. The patent family of the Mexican patent MXPA03008643 (application number MX2003PA08643) discloses process for the synthesis of the agent.

In a further embodiment of the pharmaceutical composition as herein disclosed, the pharmaceutical composition does not comprise vitamin D.

In a further embodiment of the pharmaceutical composition as herein disclosed, the pharmaceutical composition comprises the agent as the only active ingredient in the pharmaceutical composition and the agent is the distrontium salt of the acid 2-(N,N-di(carboxymethyl)amino)-3-cyano-4-carboxymethyl-5-carboxythiophene. Preferably, the pharmaceutical composition comprises the agent the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate. More preferably, the pharmaceutical composition comprises the agent the distrontium salt of the acid 2-(N,N-di(carboxymethyl)amino)-3-cyano-4-carboxymethyl-5-carboxythiophene in form of heptahydrate, a mixture of tetrahydrate/heptahydrate or a mixture octahydrate/heptahydrate.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the pharmaceutical composition comprises from 35 to 75 % w/w of the agent measured as anhydrous form in respect of the total amount of the pharmaceutical composition. The composition as herein disclosed can comprise several hydrates of the agent, so the ratio water / agent is regarding the content of the agent as it was 100% in anhydrous form.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the pharmaceutical composition comprises from 40 to 70 % w/w of the agent measured as anhydrous form in respect of the total amount of the pharmaceutical composition.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the pharmaceutical composition comprises about a 50 % w/w of the agent measured as anhydrous form in respect of the total amount of the pharmaceutical composition.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the pharmaceutical composition comprises from 2.0 to 15.0 % w/w of water content in respect of the total amount of the pharmaceutical composition or the ratio water content / agent measured as anhydrous form ranges from 3.6 to 30.5 % w/w. Preferably, the agent is the distrontium salt of the acid 2-(N,N-di(carboxymethyl)amino)-3-cyano-4-carboxymethyl-5-carboxythiophene and the pharmaceutical composition comprises from 4.0 to 14.5 % w/w of water content in respect of the total amount of the pharmaceutical composition or the ratio water content / agent measured as anhydrous form ranges from 7.0 to 29.0 % w/w. More preferably, the agent is the distrontium salt of the acid 2-(N,N-di(carboxymethyl)amino)-3-cyano-4-carboxymethyl-5-carboxythiophene and the pharmaceutical composition comprises from 8.0 to 13.0 % w/w of water content in respect of the total amount of the pharmaceutical composition or the ratio water content / agent measured as anhydrous form ranges from 15.0 to 26.0 % w/w.

The amount of water of the pharmaceutical compositions as herein disclosed was measured performing a loss on drying (LOD) using an IR halogen. For the determination of the loss on drying by infrared light the following device was used. Under GMP conditions the measurement was performed using the heat balance MA 45 Moisture Analyzer® (Sartorius AG, Göttigen, Germany). The temperature was set to 105°C. 1 gram of sample was dried for 30 min.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the D50 of the immediate release granules ranges from 140 to 800 microns and the D90 is below 1600 microns.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the D50 of the immediate release granules ranges from 200 to 600 microns and the D90 is below 1300 microns.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and wherein the D50 of the immediate release granules ranges from 300 to 500 microns and the D90 is below 1000 microns.

D50 and D90 represent the 50 percentile and the 90 percentile of the particle size distribution, respectively, as measured by the method described in European Pharmacopoeia 6.2 2.9.38. (Particle size distribution estimation by analytical sieving). That is, D50 (D90) is a value on the distribution such that 50 % (90 %) of the particles has a particle size of this value or less.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the Carr index of the granulate is below 25, preferably the Carr index of the granulate is below 22 and more preferably the Carr index of the granulate is below 20.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises at least a diluent, preferably the agent is the distrontium salt of the acid 2-(N,N-di(carboxymethyl)amino)-3-cyano-4-carboxymethyl-5-carboxythiophene and the composition comprises a total amount of diluent or diluents between a 10 and a 50 % w/w. More preferably, the agent is the distrontium salt of the acid 2-(N,N-di(carboxymethyl)amino)-3-cyano-4-carboxymethyl-5-carboxythiophene and the composition comprises a total amount of diluent or diluents between a 15 and a 35 % w/w. Even more preferably, the agent is the distrontium salt of the acid 2-(N,N-di(carboxymethyl)amino)-3-cyano-4-carboxymethyl-5-carboxythiophene and the composition comprises a total amount of diluent or diluents between a 20 and a 30 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises a diluent or diluents and the diluent or diluents being a pentahydric alcohol, an hexahydric alcohol or mixtures thereof, preferably the agent is the distrontium salt of the acid 2-(N,N-di(carboxymethyl)amino)-3-cyano-4-carboxymethyl-5-carboxythiophene and the composition comprises a diluent or diluents and the diluent or diluents being mannitol, xylitol, sorbitol or mixtures thereof. More preferably the agent is the distrontium salt of the acid 2-(N,N-di(carboxymethyl)amino)-3-cyano-4-carboxymethyl-5-carboxythiophene and the composition comprises a diluent and the diluent is mannitol.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises mannitol.

The term "diluent" as used herein is defined as an agent able to increase the bulk of a solid pharmaceutical composition, and may make a pharmaceutical dosage form containing the composition easier for the patient and/or a caregiver to handle.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises at least a binder and preferably the agent is the distrontium salt of the acid 2-(N,N-di(carboxymethyl)amino)-3-cyano-4-carboxymethyl-5-carboxythiophene and the composition comprises a total amount of binder or binders between a 1 and a 40 % w/w. More preferably the agent is the distrontium salt of the acid 2-(N,N-di(carboxymethyl)amino)-3-cyano-4-carboxymethyl-5-carboxythiophene and the composition comprises a total amount of binder or binders between a 3 and a 25 % w/w. Even more preferably, the agent is the distrontium salt of the acid 2-(N,N-di(carboxymethyl)amino)-3-cyano-4-carboxymethyl-5-carboxythiophene and the composition comprises a total amount of binder or binders between a 6 and a 15 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises a binder or binders and the binder or binders being polyvinylpyrrolidone or a polysaccharide, preferably composed by mixture of glucose and dextrose. Preferably, the agent is the distrontium salt of the acid 2-(N,N-di(carboxymethyl)amino)-3-cyano-4-carboxymethyl-5-carboxythiophene and the composition comprises a binder or binders and the binder or binders being a polysaccharide composed by mixture of glucose and dextrose. Even more preferably, the agent is the distrontium salt of the acid 2-(N,N-di(carboxymethyl)amino)-3-cyano-4-carboxymethyl-5-carboxythiophene and the composition comprises a binder and the binder is maltodextrin. In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises maltodextrin.

Maltodextrin refers to a group of short chain polysaccharide (complex) carbohydrates, which are defined as a repeating unit of a simple sugar (like glucose or dextrose). Maltodextrin is derived from a natural starch by either exposing to acid or enzymes to partially digest and break down the starch into smaller polymers.

The term "binder" as used herein is defined as an agent able to bind particles which cannot be bound only by a simple compression force. The binder may be present in the pharmaceutical composition in the form of a single compound or in the form of a mixture of compounds. Preferred binders for the compositions as herein disclosed are maltodextrin and/or polyvinylpyrrolidone.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises at least a sweetener agent. The pharmaceutical composition as herein disclosed does not have bad taste, therefore a high amount flavouring agent or sweetener to mask the taste is not needed. In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.01 and a 5 % w/w. In a further preferred embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.05 and a 2 % w/w. In most preferred embodiment, the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.1 and a 1 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises a sweetener agent or sweetener agents and the sweetener agent or sweetener agents being a non-saccharide sweetener. Preferably, the agent is the distrontium salt of the acid 2-(N,N-di(carboxymethyl)amino)-3-cyano-4-carboxymethyl-5-carboxythiophene and the composition comprises a sweetener agent and the sweetener agent is aspartame. In a further preferred embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises aspartame.

In a preferred embodiment of the pharmaceutical composition as herein disclosed, the pharmaceutical composition does not include any lubricant.

In a further embodiment the pharmaceutical composition as herein disclosed contains less than 40 % w/w of lactose, preferably less than 20 % w/w and more preferably the compositions as herein disclosed are essentially free of lactose, and even more preferably does not contain lactose.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises at least a diluent and at least a binder. In a further preferred embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises a total amount of diluent or diluents between a 10 and a 50 % w/w and the composition comprises a total amount of binder or binders between a 1 and a 40 % w/w. Even more preferred, the agent is the distrontium salt of the acid 2-(N,N-di(carboxymethyl)amino)-3-cyano-4-carboxymethyl-5-carboxythiophene and the composition comprises a total amount of diluent or diluents between a 15 and a 35 % w/w and the composition comprises a total amount of binder or binders between a 3 and a 25 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises at least a diluent and at least a sweetener agent. Preferably, the agent is the distrontium salt of the acid 2-(N,N-di(carboxymethyl)amino)-3-cyano-4-carboxymethyl-5-carboxythiophene and the composition comprises a total amount of diluent or diluents between a 10 and a 50 % w/w and the composition comprises a total amount of sweetener agent or sweetener agents between a 0.01 and a 5 % w/w. Even more preferably, the agent is the distrontium salt of the acid 2-(N,N-di(carboxymethyl)amino)-3-cyano-4-carboxymethyl-5-carboxythiophene and the composition comprises a total amount of diluent or diluents between a 15 and a 35 % w/w and the composition comprises a total amount of sweetener agent or sweetener agents between a 0.05 and a 2 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises at least a sweetener agent and at least a binder. Preferably, the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.01 and a 5 % w/w and the composition comprises a total amount of binder or binders between a 1 and a 40 % w/w. More preferably, the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.05 and a 2 % w/w and the composition comprises a total amount of binder or binders between a 3 and a 25 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the composition comprises at least a diluent, at least a binder and at least a sweetener agent. Preferably, the agent is the distrontium salt of the acid 2-(N,N-di(carboxymethyl)amino)-3-cyano-4-carboxymethyl-5-carboxythiophene and the composition comprises a total amount of diluent or diluents between a 10 and a 50 % w/w, the composition comprises a total amount of binder or binders between a 1 and a 40 % w/w and the composition comprises a total amount of sweetener agent or sweetener agents between a 0.01 and a 5 % w/w. Even more preferably, the agent is the distrontium salt of the acid 2-(N,N-di(carboxymethyl)amino)-3-cyano-4-carboxymethyl-5-carboxythiophene and the composition comprises a total amount of diluent or diluents between a 15 and a 35 % w/w, the composition comprises a total amount of binder or binders between a 3 and a 25 % w/w and the composition comprises a total amount of sweetener agent or sweetener agents between a 0.05 and a 2 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises at least a diluent and at least a binder.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises a total amount of diluent or diluents between a 10 and a 50 % w/w and the composition comprises a total amount of binder or binders between a 1 and a 40 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises a total amount of diluent or diluents between a 15 and a 35 % w/w and the composition comprises a total amount of binder or binders between a 3 and a 25 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises: from 2.0 to 15.0 % w/w of water content in respect of the total amount of the pharmaceutical composition or the ratio water content / agent measured as anhydrous form ranges from 3.6 to 30.5 % w/w; at least a diluent; and at least a binder.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises: from 2.0 to 15.0 % w/w of water content in respect of the total amount of the pharmaceutical composition or the ratio water content / agent measured as anhydrous form ranges from 3.6 to 30.5 % w/w; a total amount of diluent or diluents between a 10 and a 50 % w/w; and a total amount of binder or binders between a 1 and a 40 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises: from 2.0 to 15.0 % w/w of water content in respect of the total amount of the pharmaceutical composition or the ratio water content / agent measured as anhydrous form ranges from 3.6 to 30.5 % w/w; a total amount of diluent or diluents between a 15 and a 35 % w/w; and a total amount of binder or binders between a 3 and a 25 % w/w.

In a further embodiment of the pharmaceutical composition as herein disclosed, the agent is the distrontium salt of the acid 2-(N,N-di(carboxymethyl)amino)-3-cyano-4-carboxymethyl-5-carboxythiophene and the amount of agent measured as anhydrous form is from 1.75 to 2.25 grams.

In a further embodiment of the pharmaceutical composition as herein disclosed, the pharmaceutical composition is in the form of stick-pack.

In a further embodiment of the pharmaceutical composition as herein disclosed, the pharmaceutical composition is in the form of a stick-pack, wherein the pharmaceutical composition comprises at least the following two portions:
i) a granulate comprising the distrontium salt of the acid 2-(N,N-di(carboxymethyl)amino)-3-cyano-4-carboxymethyl-5-carboxythiophene;
ii) a stick package, wherein the stick package comprises at least a sealed laminated foil of aluminium of less than 20 microns of thickness.

A packaging of the stick-pack type, preferably with improved opening, comprises normally: a flexible film, having at least one layer, which forms a hermetically sealed tubular body with mutually opposite longitudinal film flaps, a first band provided longitudinally to said body for inside/outside sealing of said mutually opposite longitudinal flaps of the film; second sealing bands provided transversely to said body for inside/outside sealing; a sealed extension region protruding from at least one of said second sealing bands on a respective portion of at least one edge of said tubular body; and preferably a transverse preweakening incisions that are provided in longitudinal alignment with said sealed extension region, along at least one of said mutually opposite longitudinal flaps. The WO9501921 disclose stick-packs for the present application.

Preferably, the product is packaged in flexible laminate stick-packs composed of printed paper/polyethylene/aluminium foil/polyethylene. The stick-packs can be supplied in conventional cardboard boxes with Patient information Leaflets in pack sizes of more than 4 stick-packs.

In further embodiment the sealed laminated foil of aluminium of the stick package is from 5 to 16.0 microns of thickness, preferably from 7 to 12.0 microns of thickness, more preferably less than 11.0 microns of thickness, and even more preferably less than 10 microns, even yet more preferably the sealed laminated sheet of aluminium of the stick package is about 9 microns of thickness. The pharmaceutical compositions as herein disclosed showed a good stability even being packaged in a stick-pack with a thin aluminium layer.

In a further embodiment, the package comprises at least an internal layer and preferably the internal layer is of polyethylene. In a further embodiment, the package comprises at least an external layer and preferably the external layer is of polyethylene. In a further embodiment, the package comprises at least an external layer and at least an internal layer and preferably the package comprises at least a polyethylene external layer and at least a polyethylene internal layer. In a further embodiment, the package comprises at least an outer layer and preferably the outer layer is printed paper. In a further embodiment, the stick package consists essentially of printed paper foil/polyethylene foil/aluminium foil/polyethylene foil. 4 layered foils makes the formulation stable and convenient to carry.

In a further embodiment of the pharmaceutical composition as herein disclosed, the pharmaceutical compositions are manufactured by wet granulation techniques.

As used herein, the term "granulation" refers to the process of agglomerating powder particles into larger agglomerates (i.e. granules) that contain the active pharmaceutical ingredient. The term "granulation" includes wet granulation techniques. The term "wet granulation" refers to any process comprising the steps of addition of a liquid to powder starting materials, preferably kneading, and drying to yield a solid dosage form.

In a further embodiment of the pharmaceutical composition as herein disclosed, the pharmaceutical composition as herein disclosed are used for the treatment of osteoporosis, preferably for the treatment of osteoporosis in postmenopausal women to reduce the risk of vertebral and hip fractures and /or in adult men at increased risk of fracture.

The pharmaceutical compositions as herein disclosed are oral immediate release dosage forms. An immediate release dosage form as herein disclosed has to be understood as a dosage form having a dissolution performance such as 60 % or more of the agent contained in said pharmaceutical composition dissolves within 60 minutes. In a preferred embodiment, the immediate release composition as herein disclosed releases at least 80 % of the agent in 60 minutes. In another embodiment, the pharmaceutical composition as herein disclosed releases at least a 80 % of the agent in 45 min, preferably in 35 min and more preferably in 30 min. In another embodiment, the pharmaceutical composition as herein disclosed releases at least a 80 % of the agent in 30 min and at least a 95 % of the agent in 60 min. In a most preferred embodiment, the pharmaceutical composition as herein disclosed releases at least a 80 % of the agent in 15 min, and at least a 95 % of the agent in 60 min. The dissolution test for an immediate release pharmaceutical composition comprising the agent as herein disclosed is performed in the following conditions: USP Apparatus: II (Paddles). Speed (RPMs): 100. Medium: 0.1 N HCl and Phosphate sodium tribasic buffer, pH 6.8. Wavelength 306 nm. The immediate release pharmaceutical composition as herein disclosed are stable immediate release pharmaceutical composition, what it means that the dissolution profile of the pharmaceutical composition as herein disclosed when the pharmaceutical composition as herein disclosed is stored for 3 month at 40°C/75 %RH the dissolution profile of the pharmaceutical composition as herein disclosed still corresponds to an immediate release composition.

The process to manufacture the pharmaceutical composition as herein disclosed does not require any special conditions or facilities to be carried out. In addition, the process produces granules which flow without the need of adding a extragranular excipient. The pharmaceutical composition does not require the presence of colloidal silica, as for example Aerosil®, to have a good flowability.

In a further embodiment of the process to manufacture the pharmaceutical composition as herein disclosed, the binder comprises water. Preferably, the pharmaceutical composition as herein disclosed, the solvent of the binding solution consists essentially of water; even more preferably consists of water. In a further embodiment of the process to manufacture the pharmaceutical composition as herein disclosed, the binder consists of water and maltodextrin.

In a further embodiment of the process to manufacture the pharmaceutical composition as herein disclosed, in step (iv) the granulate of the previous step (ii) or (iii) is dried to an endpoint water content from 4.0 to 14.5 % w/w respect of the total amount of the pharmaceutical composition or the ratio water content / agent measured as anhydrous form ranges from 7.0 to 29.0 % w/w. Preferably, in step (iv) the granulate of the previous step (ii) or (iii) is dried to an endpoint water content from 8.0 to 13.0 % w/w in respect of the total amount of the pharmaceutical composition or the ratio water content / agent measured as anhydrous form ranges from 15.0 to 26.0 % w/w. More preferably, in step (iv) the granulate of the previous step (ii) or (iii) is dried to an endpoint water content from 2.0 to 15.0 % w/w in respect of the total amount of the pharmaceutical composition or the ratio water content / agent measured as anhydrous form is from 3.6 to 30.5 % w/w.

In a further embodiment of the process to manufacture the pharmaceutical composition as herein disclosed, in step (iv) the drying of the granulate is carried out in a fluid bed at an input temperature between 35 and 90 °C. Preferably, in step (iv) the drying of the granulate is carried out in a fluid bed at an input temperature between 40 and 80 °C. More preferably, in step (iv) the drying of the granulate is carried out in a fluid bed at an input temperature between 45 and 65 °C. Even more preferably, in step (iv) the drying of the granulate is carried out in a fluid bed at an input temperature between 50 and 60 °C. Even yet more preferably, in step (iv) the drying of the granulate is carried out in a fluid bed at an input temperature about 55 °C.

In a further embodiment of the process to manufacture the pharmaceutical composition as herein disclosed, the agent used in step (ii) is the agent the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate. In a further embodiment of the process to manufacture the pharmaceutical composition as herein disclosed, the agent used in step (ii) is the agent the distrontium salt of the acid 2-(N,N-di(carboxymethyl)amino)-3-cyano-4-carboxymethyl-5-carboxythiophene in form of heptahydrate, octahydrate or a mixture octahydrate/heptahydrate. In a further embodiment of the process to manufacture the pharmaceutical composition as herein disclosed, the agent used in step (ii) is the agent the distrontium salt of the acid 2-(N,N-di(carboxymethyl)amino)-3-cyano-4-carboxymethyl-5-carboxythiophene in form of octahydrate. In a further embodiment of the process to manufacture the pharmaceutical composition as herein disclosed, the agent used in step (ii) is the agent the distrontium salt of the acid 2-(N,N-di(carboxymethyl)amino)-3-cyano-4-carboxymethyl-5-carboxythiophene in form of heptahydrate. In a further embodiment of the process to manufacture the pharmaceutical composition as herein disclosed, the agent used in step (ii) or the distrontium salt of the acid 2-(N,N-di(carboxymethyl)amino)-3-cyano-4-carboxymethyl-5-carboxythiophene has an average particle size from 1 to 250 microns as measured by laser light diffraction.

Laser diffraction is a particle size method which uses the average relative angular intensity of scattered light. Instruments that use laser light diffraction to measure particle size have been available for many years from different manufacturers (see e.g. US 5,610,712).

The particle size determination of the agent is determined according to the following method: Apparatus: Malvern Mastersizer 2000S with sample dispersion unit. Dispersant: Isopar G. Surfactant: 5 % (W/V) phosphatidylcholines, egg and Isopar G solution. Instrument preparation: The background detect signal should be less than 100, and obscuration % value should not exceed 0.1 %. Sample preparation: Weight about 50 mg of sample into 100 ml beaker; add 2 ml of surfactant, mix and add 20 ml of dispersant. Ultrasonic for 1 minute. Pipette prepared sample into measuring cell

In a further embodiment of the process to manufacture the pharmaceutical composition as herein disclosed, the agent used in step (ii) or the distrontium salt of the acid 2-(N,N-di(carboxymethyl)amino)-3-cyano-4-carboxymethyl-5-carboxythiophene has an average particle size from 10 to 50 microns as measured by laser light diffraction.

In a further embodiment of the process to manufacture the pharmaceutical composition as herein disclosed, at least 10,000 units of pharmaceutical composition are manufactured and each unit of pharmaceutical composition comprises an amount of agent the distrontium salt of the acid 2-(N,N-di(carboxymethyl)amino)-3-cyano-4-carboxymethyl-5-carboxythiophene measured as anhydrous form from 1.75 to 2.25 grams.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

Each pharmaceutical batch of finished product must fulfil the regulatory requirements of the corresponding Medicine Agency before being released for sale or supply, such as impurities thresholds and stability data.

The pharmaceutical composition may be manufactured at laboratory scale, not necessarily following GMP and not intended for commercialization. The pharmaceutical composition may also be manufactured for validation, following GMP. A batch of a pharmaceutical composition that is manufactured for validation is called "pilot batch".

In a further embodiment of the pharmaceutical batch as herein disclosed, the agent is the distrontium salt of the acid 2-(N,N-di(carboxymethyl)amino)-3-cyano-4-carboxymethyl-5-carboxythiophene and the amount of agent measured as anhydrous form ranges from 1.75 to 2.25 grams in each unit.

In a further embodiment of the pharmaceutical batch as herein disclosed, the units are in the form of stick-pack, wherein each unit comprises at least the following two portions:
i) a granulate comprising the distrontium salt of the acid 2-(N,N-di(carboxymethyl)amino)-3-cyano-4-carboxymethyl-5-carboxythiophene;
ii) a stick package, wherein the stick package comprises at least a sealed laminated foil of aluminium.

In a further embodiment of the pharmaceutical batch as herein disclosed, the relative standard deviation value of content uniformity is less than 5 %, preferably less than 1 %.

Further aspect and embodiments of the present invention can be found in the following numbered clauses:
Clause 1.- A pharmaceutical composition in the form of immediate release granules, wherein the pharmaceutical composition comprises:
   a) an agent from 25 to 85 % w/w in respect of the total amount of the pharmaceutical composition;
   b) up to 15.7 % w/w of water content in respect of the total amount of the pharmaceutical composition or the ratio water content / agent measured as anhydrous form is up to 31.5 % w/w;
   wherein the agent is the distrontium salt of the acid 2-(N,N-di(carboxymethyl)amino)-3-cyano-4-carboxymethyl-5-carboxythiophene, a derivative or a homologue thereof; and wherein the D50 of the immediate release granules ranges from 140 to 800 microns and, advantageously, the D90 is below 1600 microns.
Clause 2.- The pharmaceutical composition according to the preceding clause, wherein the agent is the distrontium salt of the acid 2-(N,N-di(carboxymethyl)amino)-3-cyano-4-carboxymethyl-5-carboxythiophene.
Clause 3.- The pharmaceutical composition according to any one of the preceding clauses, wherein the pharmaceutical composition does not comprise vitamin D.
Clause 4.- The pharmaceutical composition according to any one of the preceding clauses, wherein the pharmaceutical composition comprises the agent as the only active ingredient in the pharmaceutical composition and the agent is the distrontium salt of the acid 2-(N,N-di(carboxymethyl)amino)-3-cyano-4-carboxymethyl-5-carboxythiophene.
Clause 5.- The pharmaceutical composition according to any one of the preceding clauses, wherein the pharmaceutical composition comprises the agent the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate.
Clause 6.- The pharmaceutical composition according to any one of the preceding clauses, wherein the pharmaceutical composition comprises the agent the distrontium salt of the acid 2-(N,N-di(carboxymethyl)amino)-3-cyano-4-carboxymethyl-5-carboxythiophene in form of heptahydrate, a mixture of tetrahydrate/heptahydrate or a mixture octahydrate/heptahydrate.
Clause 7.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the pharmaceutical composition comprises from 35 to 75 % w/w of the agent measured as anhydrous form in respect of the total amount of the pharmaceutical composition.
Clause 8.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the pharmaceutical composition comprises from 40 to 70 % w/w of the agent measured as anhydrous form in respect of the total amount of the pharmaceutical composition.
Clause 9.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the pharmaceutical composition comprises about a 50 % w/w of the agent measured as anhydrous form in respect of the total amount of the pharmaceutical composition.
Clause 10.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the pharmaceutical composition comprises from 2.0 to 15.0 % w/w of water content in respect of the total amount of the pharmaceutical composition or the ratio water content / agent measured as anhydrous form ranges from 3.6 to 30.5 % w/w.
Clause 11.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the pharmaceutical composition comprises from 4.0 to 14.5 % w/w of water content in respect of the total amount of the pharmaceutical composition or the ratio water content / agent measured as anhydrous form ranges from 7.0 to 29.0 % w/w.
Clause 12.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the pharmaceutical composition comprises from 8.0 to 13.0 % w/w of water content in respect of the total amount of the pharmaceutical composition or the ratio water content / agent measured as anhydrous form ranges from 15.0 to 26.0 % w/w.
Clause 13.- The pharmaceutical composition according to any one of the preceding clauses, wherein the agent is the distrontium salt of the acid 2-(N,N-di(carboxymethyl)amino)-3-cyano-4=carboxymethyl-5-carboxythiophene and the D50 of the immediate release granules ranges from 140 to 800 microns and the D90 is below 1600 microns.
Clause 14.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and wherein the D50 of the immediate release granules ranges from 200 to 600 microns and the D90 is below 1300 microns.
Clause 15.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and wherein the D50 of the immediate release granules ranges from 300 to 500 microns and the D90 is below 1000 microns.
Clause 16.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the Carr index of the granulate is below 25.
Clause 17.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the Carr index of the granulate is below 22.
Clause 18.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the Carr index of the granulate is below 20.
Clause 19.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises at least a diluent.
Clause 20.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 10 and a 50 % w/w.
Clause 21.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 15 and a 35 % w/w.
Clause 22.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 20 and a 30 % w/w.
Clause 23.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a diluent or diluents and the diluent or diluents being a pentahydric alcohol, an hexahydric alcohols or mixtures thereof.
Clause 24.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a diluent or diluents and the diluent or diluents being mannitol, xylitol, sorbitol or mixtures thereof.
Clause 25.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a diluent and the diluent is mannitol.
Clause 26.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises mannitol.
Clause 27.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises at least a binder.
Clause 28.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of binder or binders between a 1 and a 40 % w/w.
Clause 29.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of binder or binders between a 3 and a 25 % w/w.
Clause 30.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of binder or binders between a 6 and a 15 % w/w.
Clause 31.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a binder or binders and the binder or binders being polyvinylpyrrolidone or a polysaccharide, preferably composed by mixture of glucose and dextrose.
Clause 32.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a binder or binders and the binder or binders being polysaccharide composed by a mixture of glucose and dextrose.
Clause 33.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a binder and the binder is maltodextrin.
Clause 34.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises maltodextrin.
Clause 35.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises at least a sweetener agent.
Clause 36.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.01 and a 5 % w/w.
Clause 37.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.05 and a 2 % w/w.
Clause 38.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.1 and a 1 % w/w.
Clause 39.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a sweetener agent or sweetener agents and the sweetener agent or sweetener agents being a non-saccharide sweetener.
Clause 40.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a sweetener agent and the sweetener agent is aspartame.
Clause 41.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises aspartame.
Clause 42.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises at least a diluent and at least a binder.
Clause 43.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 10 and a 50 % w/w and the composition comprises a total amount of binder or binders between a 1 and a 40 % w/w.
Clause 44.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 15 and a 35 % w/w and the composition comprises a total amount of binder or binders between a 3 and a 25 % w/w.
Clause 45.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises at least a diluent and at least a sweetener agent.
Clause 46.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 10 and a 50 % w/w and the composition comprises a total amount of sweetener agent or sweetener agents between a 0.01 and a 5 % w/w.
Clause 47.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 15 and a 35 % w/w and the composition comprises a total amount of sweetener agent or sweetener agents between a 0.05 and a 2 % w/w.
Clause 48.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises at least a sweetener agent and at least a binder.
Clause 49.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.01 and a 5 % w/w and the composition comprises a total amount of binder or binders between a 1 and a 40 % w/w.
Clause 50.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of sweetener agent or sweetener agents between a 0.05 and a 2 % w/w and the composition comprises a total amount of binder or binders between a 3 and a 25 % w/w.
Clause 51.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises at least a diluent, at least a binder and at least a sweetener agent.
Clause 52.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 10 and a 50 % w/w, the composition comprises a total amount of binder or binders between a 1 and a 40 % w/w and and the composition comprises a total amount of sweetener agent or sweetener agents between a 0.01 and a 5 % w/w.
Clause 53.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the composition comprises a total amount of diluent or diluents between a 15 and a 35 % w/w, the composition comprises a total amount of binder or binders between a 3 and a 25 % w/w and the composition comprises a total amount of sweetener agent or sweetener agents between a 0.05 and a 2 % w/w
Clause 54.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises at least a diluent and at least a binder.
Clause 55.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises a total amount of diluent or diluents between a 10 and a 50 % w/w and the composition comprises a total amount of binder or binders between a 1 and a 40 % w/w.
Clause 56.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises a total amount of diluent or diluents between a 15 and a 35 % w/w and the composition comprises a total amount of binder or binders between a 3 and a 25 % w/w.
Clause 57.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises: from 2.0 to 15.0 % w/w of water content in respect of the total amount of the pharmaceutical composition or the ratio water content / agent measured as anhydrous form ranges from 3.6 to 30.5 % w/w; at least a diluent; and at least a binder.
Clause 58.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises: from 2.0 to 15.0 % w/w of water content in respect of the total amount of the pharmaceutical composition or the ratio water content / agent measured as anhydrous form ranges from 3.6 to 30.5 % w/w; a total amount of diluent or diluents between a 10 and a 50 % w/w; and a total amount of binder or binders between a 1 and a 40 % w/w.
Clause 59.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate and the composition comprises: from 2.0 to 15.0 % w/w of water content in respect of the total amount of the pharmaceutical composition or the ratio water content / agent measured as anhydrous form ranges from 3.6 to 30.5 % w/w; a total amount of diluent or diluents between a 15 and a 35 % w/w; and a total amount of binder or binders between a 3 and a 25 % w/w.
Clause 60.- The pharmaceutical composition according to any one of the preceding clauses, wherein the agent is the distrontium salt of the acid 2-(N,N-di(carboxymethyl)amino)-3-cyano-4-carboxymethyl-5-carboxythiophene and the amount of agent measured as anhydrous form is from 1.75 to 2.25 grams.
Clause 61.- The pharmaceutical composition according to any one of the preceding clauses or the pharmaceutical composition comprising the agent according to any one of the preceding clauses, wherein the pharmaceutical composition is in the form of stick-pack.
Clause 62.- The pharmaceutical composition according to any one of the preceding clauses, wherein the pharmaceutical composition is in the form of a stick-pack, wherein the pharmaceutical composition comprises at least the following two portions:
   i) a granulate comprising the distrontium salt of the acid 2-(N,N-di(carboxymethyl)amino)-3-cyano-4-carboxymethyl-5-carboxythiophene, preferably the granulate comprises an amount of the distrontium salt of the acid 2-(N,N-di(carboxymethyl)amino)-3-cyano-4-carboxymethyl-5-carboxythiophene measured as anhydrous form ranging from 1.75 to 2.25 grams in each stick-pack;
   ii) a stick package, wherein the stick package comprises at least a sealed laminated foil of aluminium.
Clause 63.- The pharmaceutical composition according to any one of the preceding clauses, wherein the pharmaceutical composition is in the form of a stick-pack, wherein the pharmaceutical composition comprises at least the following two portions:
   i) a granulate comprising the distrontium salt of the acid 2-(N,N-di(carboxymethyl)amino)-3-cyano-4-carboxymethyl-5-carboxythiophene;
   ii) a stick package, wherein the stick package comprises at least a sealed laminated foil of aluminium of less than 20 microns of thickness.
Clause 64.- The pharmaceutical composition according to the preceding clause, wherein the pharmaceutical compositions is manufactured by wet granulation techniques.
Clause 65.- The pharmaceutical composition according to any one of the preceding clauses for the treatment of osteoporosis, preferably for the treatment of osteoporosis in postmenopausal women to reduce the risk of vertebral and hip fractures and /or in adult men at increased risk of fracture.
Clause 66.- A process for the manufacture of a pharmaceutical composition in the form of immediate release granules according to any one of the preceding clauses, wherein the pharmaceutical composition comprises:
   a) an agent from 25 to 85 % w/w in respect of the total amount of the pharmaceutical composition;
   b) up to 15.7 % w/w of water content in respect of the total amount of the pharmaceutical composition or the ratio water content / agent measured as anhydrous form is up to 31.5 % w/w;
      wherein the agent is the distrontium salt of the acid 2-(N,N-di(carboxymethyl)amino)-3-cyano-4-carboxymethyl-5-carboxythiophene, a derivative or a homologue thereof and, advantageously, the agent is the distrontium salt of the acid 2-(N,N-di(carboxymethyl)amino)-3-cyano-4-carboxymethyl-5-carboxythiophene; and wherein the D50 of the immediate release granules ranges from 140 to 800 microns and, advantageously, the D90 is below 1600 microns;
      wherein the process for the manufacture of the pharmaceutical composition in the form of immediate release granules comprises the steps of:
      i) sieving when appropriate the intra-granular ingredients to form uniformly sized particles of intra-granular ingredients;
      ii) mixing the particles of intra-granular ingredients and the distrontium salt of the acid 2-(N,N-di(carboxymethyl)amino)-3-cyano-4-carboxymethyl-5-carboxythiophene, a derivative or a homologue thereof, preferably in a granulator-mixer; granulate the obtained mixture with at least one binder, preferably the binder comprises water and a binder agent, more preferably consists essentially of water and maltodextrin;
      iii) optionally and/or if required, performing a sieving of the granulate obtained in step (ii);
      iv) drying the granulate of the previous step (ii) or (iii) to an endpoint up to 15.7 % w/w of water content in respect of the total amount of the granulate or the ratio water content / agent measured as anhydrous form is up to 31.5 % w/w, wherein the drying is preferably carried out in a fluid bed at an input temperature between 35 and 105 °C.
Clause 67.- The process according to the preceding clause, wherein the binder comprises water.
Clause 68.- The process according to any one of the preceding process clauses, wherein the solvent of the binding solution consists essentially of water; even more preferably consists of water.
Clause 69.- The process according to any one of the preceding process clauses, wherein the binder consists of water and maltodextrin.
Clause 70.- The process according to any one of the preceding process clauses, wherein in step (iv) the granulate of the previous step (ii) or (iii) is dried to an endpoint water content from 2.0 to 15.0 % w/w in respect of the total amount of the pharmaceutical composition or the ratio water content / agent measured as anhydrous form is from 3.6 to 30.5 % w/w.
Clause 71.- The process according to any one of the preceding process clauses, wherein in step (iv) the granulate of the previous step (ii) or (iii) is dried to an endpoint water content from 4.0 to 14.5 % w/w respect of the total amount of the pharmaceutical composition or the ratio water content / agent measured as anhydrous form ranges from 7.0 to 29.0 % w/w.
Clause 72.- The process according to any one of the preceding process clauses, wherein in step (iv) the granulate of the previous step (ii) or (iii) is dried to an endpoint water content from 8.0 to 13.0 % w/w in respect of the total amount of the pharmaceutical composition or the ratio water content / agent measured as anhydrous form ranges from 15.0 to 26.0 % w/w.
Clause 73.- The process according to any one of the preceding process clauses, wherein in step (iv) the drying of the granulate is carried out in a fluid bed at an input temperature between 35 and 90 °C.
Clause 74.- The process according to any one of the preceding process clauses, wherein in step (iv) the drying of the granulate is carried out in a fluid bed at an input temperature between 40 and 80 °C.
Clause 75.- The process according to any one of the preceding process clauses, wherein in step (iv) the drying of the granulate is carried out in a fluid bed at an input temperature between 45 and 65 °C.
Clause 76.- The process according to any one of the preceding process clauses, wherein in step (iv) the drying of the granulate is carried out in a fluid bed at an input temperature between 50 and 60 °C.
Clause 77.- The process according to any one of the preceding process clauses, wherein in step (iv) the drying of the granulate is carried out in a fluid bed at an input temperature about 55 °C.
Clause 78.- The process according to any one of the preceding process clauses, wherein the agent used in step (ii) is the agent the distrontium salt of the acid 2-(N,N-di(carboxymethyl)amino)-3-cyano-4-carboxymethyl-5-carboxythiophene in form of tetrahydrate, heptahydrate, octahydrate, a mixture of tetrahydrate/heptahydrate or a mixture octahydrate/heptahydrate.
Clause 79.- The process according to any one of the preceding process clauses, wherein the agent used in step (ii) is the agent the distrontium salt of the acid 2-(N,N-di(carboxymethyl)amino)-3-cyano-4-carboxymethyl-5-carboxythiophene in form of heptahydrate, octahydrate or a mixture octahydrate/heptahydrate.
Clause 80.- The process according to any one of the preceding process clauses, wherein the agent used in step (ii) or the distrontium salt of the acid 2-(N,N-di(carboxymethyl)amino)-3-cyano-4-carboxymethyl-5-carboxythiophene has an average particle size from 1 to 250 microns as measured by laser light diffraction.
Clause 81.- The process according to any one of the preceding process clauses, wherein the agent used in step (ii) or the distrontium salt of the acid 2-(N,N-di(carboxymethyl)amino)-3-cyano-4-carboxymethyl-5-carboxythiophene has an average particle size from 10 to 50 microns as measured by laser light diffraction.
Clause 82.- The process according to any one of the preceding process clauses, wherein at least 10,000 units of pharmaceutical composition are manufactured and each unit of pharmaceutical composition comprises an amount of agent the distrontium salt of the acid 2-(N,N-di(carboxymethyl)amino)-3-cyano-4-carboxymethyl-5-carboxythiophene measured as anhydrous form from 1.75 to 2.25 grams.
Clause 82.- The pharmaceutical composition according to any one of the preceding product clauses manufactured by any one of the processes as defined in any one of the preceding process clauses.
Clause 83.- A pharmaceutical batch of at least 10,000 units of the pharmaceutical composition according to any one of the preceding product clauses.
Clause 84.- The pharmaceutical batch according to the preceding clause, wherein the agent is the distrontium salt of the acid 2-(N,N-di(carboxymethyl)amino)-3-cyano-4-carboxymethyl-5-carboxythiophene and the amount of agent measured as anhydrous form ranges from 1.75 to 2.25 grams in each unit.
Clause 85.- The pharmaceutical batch according to the preceding clause, where the units are in the form of stick-pack, wherein each unit comprises at least the following two portions:
   i) a granulate comprising the distrontium salt of the acid 2-(N,N-di(carboxymethyl)amino)-3-cyano-4-carboxymethyl-5-carboxythiophene;
   ii) a stick package, wherein the stick package comprises at least a sealed laminated foil of aluminium.
Clause 85.- The pharmaceutical batch according to any one of the preceding three clauses, wherein the relative standard deviation value of content uniformity is less than 5 %, preferably less than 1 %.
Clause 86.- A cardboard cartons with patient information leaflets in pack sizes of at least 4 units of pharmaceutical composition, preferably packaged in stick-packs, as defined in any one of the pharmaceutical composition clauses.

Preferred combinations of the above clauses are as follows:
Clause 87:, 1, 5, 10
Clause 88:, 1, 5, 10, 13
Clause 89:, 1, 5, 10, 14
Clause 90, 1, 5, 10, 15
Clause 91, 1, 5, 10, 16
Clause 92, 1, 5, 10, 13, 16
Clause 93, 1, 5, 10, 14, 16
Clause 94, 1, 5, 10, 15, 16
Clause 95, 1, 5, 10, 42
Clause 96, 1, 5, 10, 13, 42
Clause 97, 1, 5, 10, 14, 42
Clause 98, 1, 5, 10, 15, 42
Clause 99, 1, 5, 10, 16, 42
Clause 100, 1, 5, 10, 13, 16, 42
Clause 101, 1, 5, 10, 14, 16, 42
Clause 102, 1, 5, 10, 15, 16, 42
Clause 103, 1, 5, 10, 43
Clause 104, 1, 5, 10, 13, 43
Clause 105, 1, 5, 10, 14, 43
Clause 106, 1, 5, 10, 15, 43
Clause 107, 1,5, 10, 16,43
Clause 108, 1, 5, 10, 13, 16, 43
Clause 109, 1, 5, 10, 14, 16, 43
Clause 110, 1, 5, 10, 15, 16, 43
Clause 111, 1, 5, 10, 52
Clause 112, 1, 5, 10, 13, 52
Clause 113, 1, 5, 10, 14, 52
Clause 114, 1, 5, 10, 15, 52
Clause 115, 1, 5, 10, 16, 52
Clause 116, 1, 5, 10, 13, 16, 52
Clause 117, 1, 5, 10, 14, 16, 52
Clause 118, 1, 5, 10, 15, 16, 52
Clause 119, 1, 5, 10, 60
Clause 120, 1, 5, 10, 13, 60
Clause 121, 1,5, 10, 14,60
Clause 122, 1, 5, 10, 15, 60
Clause 123, 1,5, 10, 16,60
Clause 124, 1, 5, 10, 13, 16, 60
Clause 125, 1, 5, 10, 14, 16, 60
Clause 126, 1, 5, 10, 15, 16, 60
Clause 127, 1, 5, 10, 42, 60
Clause 128, 1, 5, 10, 13, 42, 60
Clause 129, 1, 5, 10, 14, 42, 60
Clause 130, 1, 5, 10, 15, 42, 60
Clause 131, 1, 5, 10, 16, 42, 60
Clause 132, 1, 5, 10, 13, 16, 42, 60
Clause 133, 1, 5, 10, 14, 16, 42, 60
Clause 134, 1, 5, 10, 15, 16, 42, 60
Clause 135, 1, 5, 10, 43, 60
Clause 136, 1, 5, 10, 13, 43, 60
Clause 137, 1, 5, 10, 14, 43, 60
Clause 138, 1, 5, 10, 15, 43, 60
Clause 139, 1, 5, 10, 16, 43, 60
Clause 140, 1, 5, 10, 13, 16, 43, 60
Clause 141, 1, 5, 10, 14, 16, 43, 60
Clause 142, 1, 5, 10, 15, 16, 43, 60
Clause 143, 1, 5, 10, 52, 60
Clause 144, 1, 5, 10, 13, 52, 60
Clause 145, 1, 5, 10, 14, 52, 60
Clause 146, 1, 5, 10, 15, 52, 60
Clause 147, 1, 5, 10, 16, 52, 60
Clause 148, 1, 5, 10, 13, 16, 52, 60
Clause 149, 1, 5, 10, 14, 16, 52, 60
Clause 150, 1, 5, 10, 15, 16, 52, 60
Clause 151, 1,5, 10,61
Clause 152, 1,5, 10, 13,61
Clause 153, 1,5, 10, 14,61
Clause 154, 1,5, 10, 15,61
Clause 155, 1,5, 10, 16,61
Clause 156, 1, 5, 10, 13, 16, 61
Clause 157, 1, 5, 10, 14, 16, 61
Clause 158, 1, 5, 10, 15, 16, 61
Clause 159, 1, 5, 10, 42, 61
Clause 160, 1, 5, 10, 13, 42, 61
Clause 161, 1,5, 10, 14,42,61
Clause 162, 1, 5, 10, 15, 42, 61
Clause 163, 1, 5, 10, 16, 42, 61
Clause 164, 1, 5, 10, 13, 16, 42, 61
Clause 165, 1, 5, 10, 14, 16, 42, 61
Clause 166, 1, 5, 10, 15, 16, 42, 61
Clause 167, 1,5, 10,43,61
Clause 168, 1, 5, 10, 13, 43, 61
Clause 169, 1, 5, 10, 14, 43, 61
Clause 170, 1, 5, 10, 15, 43, 61
Clause 171, 1, 5, 10, 16, 43, 61
Clause 172, 1, 5, 10, 13, 16, 43, 61
Clause 173, 1, 5, 10, 14, 16, 43, 61
Clause 174, 1, 5, 10, 15, 16, 43, 61
Clause 175, 1, 5, 10, 52, 61
Clause 176, 1, 5, 10, 13, 52, 61
Clause 177, 1, 5, 10, 14, 52, 61
Clause 178, 1, 5, 10, 15, 52, 61
Clause 179, 1, 5, 10, 16, 52, 61
Clause 180, 1, 5, 10, 13, 16, 52, 61
Clause 181, 1, 5, 10, 14, 16, 52, 61
Clause 182, 1, 5, 10, 15, 16, 52, 61
Clause 183, 1, 5, 10, 60, 61
Clause 184, 1, 5, 10, 13, 60, 61
Clause 185, 1, 5, 10, 14, 60, 61
Clause 186, 1, 5, 10, 15, 60, 61
Clause 187, 1, 5, 10, 16, 60, 61
Clause 188, 1, 5, 10, 13, 16, 60, 61
Clause 189, 1, 5, 10, 14, 16, 60, 61
Clause 190, 1, 5, 10, 15, 16, 60, 61
Clause 191, 1, 5, 10, 42, 60, 61
Clause 192, 1, 5, 10, 13, 42, 60, 61
Clause 193, 1, 5, 10, 14, 42, 60, 61
Clause 194, 1, 5, 10, 15, 42, 60, 61
Clause 195, 1, 5, 10, 16, 42, 60, 61
Clause 196, 1, 5, 10, 13, 16, 42, 60, 61
Clause 197, 1, 5, 10, 14, 16, 42, 60, 61
Clause 198, 1, 5, 10, 15, 16, 42, 60, 61
Clause 199, 1, 5, 10, 43, 60, 61
Clause 200, 1, 5, 10, 13, 43, 60, 61
Clause 201, 1, 5, 10, 14, 43, 60, 61
Clause 202, 1, 5, 10, 15, 43, 60, 61
Clause 203, 1, 5, 10, 16, 43, 60, 61
Clause 204, 1, 5, 10, 13, 16, 43, 60, 61
Clause 205, 1, 5, 10, 14, 16, 43, 60, 61
Clause 206, 1, 5, 10, 15, 16, 43, 60, 61
Clause 207, 1,5, 10, 52, 60, 61
Clause 208, 1, 5, 10, 13, 52, 60, 61
Clause 209, 1, 5, 10, 14, 52, 60, 61
Clause 210, 1, 5, 10, 15, 52, 60, 61
Clause 211, 1, 5, 10, 16, 52, 60, 61
Clause 212, 1, 5, 10, 13, 16, 52, 60, 61
Clause 213, 1, 5, 10, 14, 16, 52, 60, 61
Clause 214, 1, 5, 10, 15, 16, 52, 60, 61
Clause 215, 1, 5, 10, 64
Clause 216, 1, 5, 10, 13, 64
Clause 217, 1, 5, 10, 14, 64
Clause 218, 1, 5, 10, 15, 64
Clause 219, 1, 5, 10, 16, 64
Clause 220, 1, 5, 10, 13, 16, 64
Clause 221, 1, 5, 10, 14, 16, 64
Clause 222, 1, 5, 10, 15, 16, 64
Clause 223, 1, 5, 10, 42, 64
Clause 224, 1, 5, 10, 13, 42, 64
Clause 225, 1, 5, 10, 14, 42, 64
Clause 226, 1, 5, 10, 15, 42, 64
Clause 227, 1,5, 10, 16,42,64
Clause 228, 1, 5, 10, 13, 16, 42, 64
Clause 229, 1, 5, 10, 14, 16, 42, 64
Clause 230, 1, 5, 10, 15, 16, 42, 64
Clause 231, 1,5, 10, 43, 64
Clause 232, 1, 5, 10, 13, 43, 64
Clause 233, 1, 5, 10, 14, 43, 64
Clause 234, 1, 5, 10, 15, 43, 64
Clause 235, 1, 5, 10, 16, 43, 64
Clause 236, 1, 5, 10, 13, 16, 43, 64
Clause 237, 1, 5, 10, 14, 16, 43, 64
Clause 238, 1, 5, 10, 15, 16, 43, 64
Clause 239, 1, 5, 10, 52, 64
Clause 240, 1, 5, 10, 13, 52, 64
Clause 241, 1, 5, 10, 14, 52, 64
Clause 242, 1, 5, 10, 15, 52, 64
Clause 243, 1, 5, 10, 16, 52, 64
Clause 244, 1, 5, 10, 13, 16, 52, 64
Clause 245, 1, 5, 10, 14, 16, 52, 64
Clause 246, 1, 5, 10, 15, 16, 52, 64
Clause 247, 1, 5, 10, 60, 64
Clause 248, 1, 5, 10, 13, 60, 64
Clause 249, 1, 5, 10, 14, 60, 64
Clause 250, 1, 5, 10, 15, 60, 64
Clause 251, 1, 5, 10, 16, 60, 64
Clause 252, 1, 5, 10, 13, 16, 60, 64
Clause 253, 1, 5, 10, 14, 16, 60, 64
Clause 254, 1, 5, 10, 15, 16, 60, 64
Clause 255, 1, 5, 10, 42, 60, 64
Clause 256, 1, 5, 10, 13, 42, 60, 64
Clause 257, 1, 5, 10, 14, 42, 60, 64
Clause 258, 1, 5, 10, 15, 42, 60, 64
Clause 259, 1, 5, 10, 16, 42, 60, 64
Clause 260, 1, 5, 10, 13, 16, 42, 60, 64
Clause 261, 1, 5, 10, 14, 16, 42, 60, 64
Clause 262, 1, 5, 10, 15, 16, 42, 60, 64
Clause 263, 1, 5, 10, 43, 60, 64
Clause 264, 1, 5, 10, 13, 43, 60, 64
Clause 265, 1, 5, 10, 14, 43, 60, 64
Clause 266, 1, 5, 10, 15, 43, 60, 64
Clause 267, 1, 5, 10, 16, 43, 60, 64
Clause 268, 1, 5, 10, 13, 16, 43, 60, 64
Clause 269, 1, 5, 10, 14, 16, 43, 60, 64
Clause 270, 1, 5, 10, 15, 16, 43, 60, 64
Clause 271, 1, 5, 10, 52, 60, 64
Clause 272, 1, 5, 10, 13, 52, 60, 64
Clause 273, 1, 5, 10, 14, 52, 60, 64
Clause 274, 1, 5, 10, 15, 52, 60, 64
Clause 275, 1, 5, 10, 16, 52, 60, 64
Clause 276, 1, 5, 10, 13, 16, 52, 60, 64
Clause 277, 1, 5, 10, 14, 16, 52, 60, 64
Clause 278, 1, 5, 10, 15, 16, 52, 60, 64
Clause 279, 1, 5, 10, 61, 64
Clause 280, 1, 5, 10, 13, 61, 64
Clause 281, 1,5, 10, 14,61,64
Clause 282, 1, 5, 10, 15, 61, 64
Clause 283, 1, 5, 10, 16, 61, 64
Clause 284, 1, 5, 10, 13, 16, 61, 64
Clause 285, 1, 5, 10, 14, 16, 61, 64
Clause 286, 1, 5, 10, 15, 16, 61, 64
Clause 287, 1,5, 10, 42, 61, 64
Clause 288, 1, 5, 10, 13, 42, 61, 64
Clause 289, 1, 5, 10, 14, 42, 61, 64
Clause 290, 1, 5, 10, 15, 42, 61, 64
Clause 291, 1,5, 10, 16, 42, 61, 64
Clause 292, 1, 5, 10, 13, 16, 42, 61, 64
Clause 293, 1, 5, 10, 14, 16, 42, 61, 64
Clause 294, 1, 5, 10, 15, 16, 42, 61, 64
Clause 295, 1, 5, 10, 43, 61, 64
Clause 296, 1, 5, 10, 13, 43, 61, 64
Clause 297, 1, 5, 10, 14, 43, 61, 64
Clause 298, 1, 5, 10, 15, 43, 61, 64
Clause 299, 1, 5, 10, 16, 43, 61, 64
Clause 300, 1, 5, 10, 13, 16, 43, 61, 64
Clause 301, 1, 5, 10, 14, 16, 43, 61, 64
Clause 302, 1, 5, 10, 15, 16, 43, 61, 64
Clause 303, 1, 5, 10, 52, 61, 64
Clause 304, 1, 5, 10, 13, 52, 61, 64
Clause 305, 1, 5, 10, 14, 52, 61, 64
Clause 306, 1, 5, 10, 15, 52, 61, 64
Clause 307, 1, 5, 10, 16, 52, 61, 64
Clause 308, 1, 5, 10, 13, 16, 52, 61, 64
Clause 309, 1, 5, 10, 14, 16, 52, 61, 64
Clause 310, 1, 5, 10, 15, 16, 52, 61, 64
Clause 311, 1, 5, 10, 60, 61, 64
Clause 312, 1, 5, 10, 13, 60, 61, 64
Clause 313, 1,5, 10, 14,60,61,64
Clause 314, 1, 5, 10, 15, 60, 61, 64
Clause 315, 1, 5, 10, 16, 60, 61, 64
Clause 316, 1, 5, 10, 13, 16, 60, 61, 64
Clause 317, 1, 5, 10, 14, 16, 60, 61, 64
Clause 318, 1, 5, 10, 15, 16, 60, 61, 64
Clause 319, 1, 5, 10, 42, 60, 61, 64
Clause 320, 1, 5, 10, 13, 42, 60, 61, 64
Clause 321, 1, 5, 10, 14, 42, 60, 61, 64
Clause 322, 1, 5, 10, 15, 42, 60, 61, 64
Clause 323, 1, 5, 10, 16, 42, 60, 61, 64
Clause 324, 1, 5, 10, 13, 16, 42, 60, 61, 64
Clause 325, 1, 5, 10, 14, 16, 42, 60, 61, 64
Clause 326, 1, 5, 10, 15, 16, 42, 60, 61, 64
Clause 327, 1, 5, 10, 43, 60, 61, 64
Clause 328, 1, 5, 10, 13, 43, 60, 61, 64
Clause 329, 1, 5, 10, 14, 43, 60, 61, 64
Clause 330, 1, 5, 10, 15, 43, 60, 61, 64
Clause 331, 1, 5, 10, 16, 43, 60, 61, 64
Clause 332, 1, 5, 10, 13, 16, 43, 60, 61, 64
Clause 333, 1, 5, 10, 14, 16, 43, 60, 61, 64
Clause 334, 1, 5, 10, 15, 16, 43, 60, 61, 64
Clause 335, 1, 5, 10, 52, 60, 61, 64
Clause 336, 1, 5, 10, 13, 52, 60, 61, 64
Clause 337, 1, 5, 10, 14, 52, 60, 61, 64
Clause 338, 1, 5, 10, 15, 52, 60, 61, 64
Clause 339, 1, 5, 10, 16, 52, 60, 61, 64
Clause 340, 1, 5, 10, 13, 16, 52, 60, 61, 64
Clause 341, 1, 5, 10, 14, 16, 52, 60, 61, 64
Clause 342, 1, 5, 10, 15, 16, 52, 60, 61, 64

All percentages, parts and ratios herein disclosed are by weight unless specifically noted otherwise. As used herein, the term "about" refers preferably to a range that is +-10 %, preferably +-5 %, or more preferably the value with which the term is associated.

### EXAMPLES

### Example 1

This example corresponds to a wet granulation. Batch size of 5 Kg of final granulate. Table 1.

| Batch 12018 | mg / stick-pack |
|---|---|
| The agent (measured as anhydrous) | 2000 |
| Aspartame | 20 |
| Maltodextrin | 400 |
| Mannitol | 948 |
| TOTAL DRY WEIGHT | 3368 |

1.- The agent, maltodextrin, mannitol and aspartame through a 1 mm screen were independently sifted.
2.- Preparation of binding solution: 75 % of the total amount of maltodextrin in the quantity of water required for granulation was dissolved in a tank. The mixture was stirred for 15 min at 5000 rpm with an Ultra-turrax.
3.- It was blended for 5 min at 200 rpm the agent, and 25 % of the total amount of maltodextrin, mannitol and aspartame.
4.- It was granulated the blend obtained in point 3 by adding the binding solution prepared in point 2. Granulation conditions are impeller speed 100 rpm and chopper 600 rpm for 11 minutes.
5.- It was continued with the granulation process for 2 minutes with impeller at 200 rpm and chopper at 1500 rpm.
6.- It was dried in a fluid bed machine until obtain a water content of less than 8 % w/w in respect of the total amount of the granulate. Fluid bed conditions used are inlet temperature 45-55°C; inlet air flow 70-150 m³/h; inlet relative humidity < 40 %.
7.- It was sifted the final dry granule obtained in point 5 through a 1.25 mm screen.

Granulometric distribution of the pharmaceutical compositions as herein disclosed is measured according to the method described in European Pharmacopoeia 6.2 2.9.38. (Particle size distribution estimation by analytical sieving). It was tared each test sieve to the nearest 0.1 g. It was placed an accurately weighed quantity of test sample on the top (coarsest) sieve, and replaced the lid. It was agitated the nest of sieves for 5 min, and then carefully removed each sieve from the nest without loss of material. It was reweighted each sieve, and determined the mass of material on each one. It was determined the mass of material in the collecting pan in a similar manner.

**Table 2. Particle size sieving results.**

| particle size (µm) | Retained (%) | Accumulated (%) |
|---|---|---|
| > 1000 | 0.72 | 100.00 |
| 850-1000 | 1.95 | 98.79 |
| 710-850 | 3.82 | 93.65 |
| 500-710 | 10.43 | 85.16 |
| 425-500 | 5.77 | 68.31 |
| 355-425 | 9.47 | 55.74 |
| 250-355 | 13.64 | 41.53 |
| 150-250 | 11.80 | 23.74 |
| 90-150 | 5.69 | 9.62 |
| 45-90 | 2.90 | 3.47 |
| < 45 | 0.46 | 0.46 |

**Table 3. Carr's index**

| Bulk density g/ml | Carr Index |
|---|---|
| 0.715 | below 23 |

**Table 4. Uniformity of weight of stick-packs during filling process:**

| stick-pack n° | Beginning | Middle | End |
|---|---|---|---|
| SD | 0.06 g | 0.09 g | 0.08 g |
| RSD | 1.28 % | 1.98 % | 1.71 % |

**Table 5. Dissolution profile at pH=6.8**

| Time (min) | Average % dissolved |
|---|---|
| 0 | 0 |
| 5 | 62 |
| 15 | 94 |
| 30 | 98 |

Stabilities studies were performed at 40 °C/75 %HR and good results were obtained.

### Example 2.

This example corresponds to a wet granulation. Batch size of 5 Kg of final granulate.

**Table 6.**

| Batch 19 | % as to dry matter | mg/stick-pack | 150 stick-packs |
|---|---|---|---|
| The agent ⁽¹⁾ | 58.14 % | 2000.00 mg | 300.00 g |
| PVP K30 | 5.81 % | 200.00 mg | 30.00 g |
| Colloidal silica | 1.16 % | 40.00 mg | 6.00 g |
| EMDEX® | 34.69 % | 1193.34 mg | 179.00 g |
| Sucralose | 0.19 % | 6.66 mg | 1.00 g |
| Purified water | -- | -- | -- |
| | | 4000 g (3440 g as dry matter) | 600.0 g |

| | | | |
|---|---|---|---|
| ⁽¹⁾ Measured as anhydrous form. | | | |

### Manufacturing process:

1.- It was sifted independently the agent, PVP K30, EMDEX®, sucralose and colloidal silica through a 1 mm screen.
2.- It was mixed the agent with 50 % of EMDEX® and mix for 5 minutes. Impeller speed: 600 rpm and chopper off.
3.- Preparation of binding solution: It was dissolved in a beaker PVP K30 in the quantity of water required for granulation.
4.- It was granulated the blend obtained in point 2 by adding binding solution prepared in step 3 for 56 seconds. Granulation conditions are impeller speed: 300 rpm and chopper off for 2.5 minutes.
5.- It was dried in a fluid bed machine until it was obtained a water content of less than 7 %.
6.- It was mixed the rest of the components (EMDEX® 50 %, colloidal silica and sucralose) with the granulation obtained in point 5.
7.- Dosification of the blend into stick-packs to a theoretical net weight of 4.000 g / stick-pack ± 5 %.

**Table 7. Weight uniformity results of automatically filled stick-packs.**

| stick-pack n° | Beginning | Middle | End |
|---|---|---|---|
| SD | 0.0693 g | 0.0750 g | 0.0602 g |
| RSD | 2.4 % | 2.5 % | 2.1 % |

### Example 3 - Comparative Example

**Table 8. This batch corresponds to a dry mixture.**

| BATCH 8 | % | mg/stick-pack | 75 stick-packs |
|---|---|---|---|
| The agent as octahydrate⁽¹⁾ | 64.00 % | 2560.00 mg | 192.00 g |
| Colloidal silica | 3.00 % | 122.00 mg | 9.00 g |
| EMDEX® | 32.5 % | 1300.00 mg | 97.50 g |
| Aspartame | 0.50 % | 20.00 mg | 1.50 g |
| | 100 % | 4000.00 mg | 300.00 g |

| | | | |
|---|---|---|---|
| ⁽¹⁾ Equivalent to 2.0 g of the agent in anhydrous form. | | | |

### Manufacturing process:

1.- It was sifted independently the agent, aspartame, EMDEX® and colloidal silica powder through a 1 mm mesh screen.
2.- It was mixed the sifted material for 25 min at 20 rpm in a blender.
3.- Dosification of the blend into stick-packs to a theoretical net weight of 4.000 g / stick-pack ± 5 %.

**Table 9. Weight uniformity for batch 8 in stick-packs 6.**

| N° | Empty stick-pack | Start | Middle | End |
|---|---|---|---|---|
| SD | 0.0109 g | 0.0884 g | 0.2523 g | 0.0752 g |
| RSD | 1.02 % | 3.77 % | 11.67 % | 3.29 % |

**Table 10. Dissolution profile of batch 8 at pH 6.8**

| Time (min) | Average % dissolved |
|---|---|
| 0 | 0 |
| 10 | 52 |
| 15 | 68 |
| 30 | 75 |
| 60 | 78 |

In all the examples the agent is the distrontium salt of the acid 2-(N,N-di(carboxymethyl)amino)-3-cyano-4-carboxymethyl-5-carboxythiophene.

## Claims

1. A pharmaceutical composition in the form of immediate release granules, wherein the pharmaceutical composition comprises:
a) an agent from 25 to 85 % w/w in respect of the total amount of the pharmaceutical composition;
b) up to 15.7 % w/w of water content in respect of the total amount of the pharmaceutical composition or the ratio water content / agent measured as anhydrous form is up to 31.5 % w/w;
wherein the agent is the distrontium salt of the acid 2-(N,N-di(carboxymethyl)amino)-3-cyano-4-carboxymethyl-5-carboxythiophene,
and wherein the D50 of the immediate release granules ranges from 140 to 800 microns.

2. The pharmaceutical composition according to the preceding claim, wherein: the pharmaceutical composition comprises about a 50 % w/w of the agent measured as anhydrous form in respect of the total amount of the pharmaceutical composition; the agent is the only active ingredient in the pharmaceutical composition in form of tetrahydrate, heptahydrate, octahydrate, a mixture of tetrahydrate/heptahydrate or a mixture octahydrate/heptahydrate.

3. The pharmaceutical composition according to any one of the preceding claims, wherein the agent is the distrontium salt of the acid 2-(N,N-di(carboxymethyl)amino)-3-cyano-4-carboxymethyl-5-carboxythiophene and the pharmaceutical composition comprises from 2.0 to 15.0 % w/w of water content in respect of the total amount of the pharmaceutical composition, the ratio water content / agent measured as anhydrous form ranges from 3.6 to 30.5 % w/w, the pharmaceutical composition comprises from 8.0 to 13.0 % w/w of water content in respect of the total amount of the pharmaceutical composition or the ratio water content / agent measured as anhydrous form ranges from 15.0 to 26.0 % w/w.

4. The pharmaceutical composition according to any one of the preceding claims, wherein the agent is the distrontium salt of the acid 2-(N,N-di(carboxymethyl)amino)-3-cyano-4-carboxymethyl-5-carboxythiophene and the D50 of the immediate release granules ranges from 140 to 800 microns and the D90 is below 1600 microns, the D50 of the immediate release granules ranges from 300 to 500 microns and the D90 is below 1000 microns or the Carr index of the granulate is below 25.

5. The pharmaceutical composition according to any one of the preceding claims, wherein the agent is the distrontium salt of the acid 2-(N,N-di(carboxymethyl)amino)-3-cyano-4-carboxymethyl-5-carboxythiophene and the composition comprises: at least a diluent and at least a binder; or a total amount of diluent or diluents between a 15 and a 35 % w/w and the composition comprises a total amount of binder or binders between a 3 and a 25 % w/w.

6. The pharmaceutical composition according to anyone of the preceding claims, wherein the agent is the distrontium salt of the acid 2-(N,N-di(carboxymethyl)amino)-3-cyano-4-carboxymethyl-5-carboxythiophene and the composition comprises: at least a diluent, at least a binder and at least a sweetener agent; or a total amount of diluent or diluents between a 15 and a 35 % w/w, a total amount of binder or binders between a 3 and a 25 % w/w and a total amount of sweetener agent or sweetener agents between a 0.05 and a 2 % w/w

7. The pharmaceutical composition according to any one of the preceding claims, wherein the agent is the distrontium salt of the acid 2-(N,N-di(carboxymethyl)amino)-3-cyano-4-carboxymethyl-5-carboxythiophene and the amount of agent measured as anhydrous form is from 1.75 to 2.25 grams and/or the pharmaceutical compositions are manufactured by wet granulation techniques.

8. The pharmaceutical composition according to any one of the preceding claims wherein the pharmaceutical composition is in the form of stick-pack, optionally the pharmaceutical composition comprises at least the following two portions:
i) a granulate comprising the distrontium salt of the acid 2-(N,N-di(carboxymethyl)amino)-3-cyano-4-carboxymethyl-5-carboxythiophene, preferably the granulate comprises an amount of the distrontium salt of the acid 2-(N,N-di(carboxymethyl)amino)-3-cyano-4-carboxymethyl-5-carboxythiophene measured as anhydrous form ranging from 1.75 to 2.25 grams in each stick-pack;
ii) a stick package, wherein the stick package comprises at least a sealed laminated foil of aluminium, wherein preferably the package comprises a sealed laminated foil of aluminium of less than 20 microns of thickness.

9. A cardboard cartons with patient information leaflets in pack sizes of at least 4 units of pharmaceutical composition, preferably packaged in stick-packs, according to any one of the preceding pharmaceutical composition claims.

10. A process for the manufacture of a pharmaceutical composition in the form of immediate release granules according to any one of the preceding claims, wherein the pharmaceutical composition comprises:
a) an agent from 25 to 85 % w/w in respect of the total amount of the pharmaceutical composition;
b) up to 15.7 % w/w of water content in respect of the total amount of the pharmaceutical composition or the ratio water content / agent measured as anhydrous form is up to 31.5 % w/w;
wherein the agent is the distrontium salt of the acid 2-(N,N-di(carboxymethyl)amino)-3-cyano-4-carboxymethyl-5-carboxythiophene, and wherein the D50 of the immediate release granules ranges from 140 to 800 microns;
wherein the process for the manufacture of the pharmaceutical composition in the form of immediate release granules comprises the steps of:
i) sieving when appropriate the intra-granular ingredients to form uniformly sized particles of intra-granular ingredients;
ii) mixing the particles of intra-granular ingredients and the distrontium salt of the acid 2-(N,N-di(carboxymethyl)amino)-3-cyano-4-carboxymethyl-5-carboxythiophene, a derivative or a homologue thereof, preferably in a granulator-mixer; granulate the obtained mixture with at least one binder, preferably the binder comprises water and a binder agent, more preferably consists essentially of water and maltodextrin;
iii) optionally and/or if required, performing a sieving of the granulate obtained in step (ii);
iv) drying the granulate of the previous step (ii) or (iii) to an endpoint up to 15.7 % w/w of water content in respect of the total amount of the granulate or the ratio water content / agent measured as anhydrous form is up to 31.5 % w/w, wherein the drying is preferably carried out in a fluid bed at an input temperature between 35 and 105 °C.

11. The process according to the preceding process claims, wherein in step (iv) the granulate of the previous step (ii) or (iii) is dried to an endpoint water content from 2.0 to 15.0 % w/w in respect of the total amount of the pharmaceutical composition or the ratio water content / agent measured as anhydrous form is from 3.6 to 30.5 % w/w.

12. The process according to any one of the preceding process claims, wherein the agent used in step (ii) or the distrontium salt of the acid 2-(N,N-di(carboxymethyl)amino)-3-cyano-4-carboxymethyl-5-carboxythiophene has an average particle size from 10 to 50 microns as measured by laser light diffraction.

13. The process according to any one of the preceding process claims, wherein at least 10,000 units of pharmaceutical composition are manufactured and each unit of pharmaceutical composition comprises an amount of agent the distrontium salt of the acid 2-(N,N-di(carboxymethyl)amino)-3-cyano-4-carboxymethyl-5-carboxythiophene measured as anhydrous form from 1.75 to 2.25 grams.

14. A pharmaceutical batch of at least 10,000 units of the pharmaceutical composition according to any one of the preceding product claims and the amount of agent measured as anhydrous form ranges from 1.75 to 2.25 grams in each unit.

15. The pharmaceutical batch according to the preceding claim, where the units are in the form of stick-pack, wherein each unit comprises at least the following two portions:
i) a granulate comprising the distrontium salt of the acid 2-(N,N-di(carboxymethyl)amino)-3-cyano-4-carboxymethyl-5-carboxythiophene;
ii) a stick package, wherein the stick package comprises at least a sealed laminated foil of aluminium, preferably of less than 20 microns of thickness.

## Patentansprüche

1. Eine pharmazeutische Zusammensetzung in Form von Granulaten zur unmittelbaren Freisetzung, wobei die pharmazeutische Zusammensetzung umfasst:
a) einen Wirkstoff von 25 bis 85 % (w/w) in Bezug auf die Gesamtmenge der pharmazeutischen Zusammensetzung;
b) bis zu 15,7 % (w/w) Wassergehalt in Bezug auf die Gesamtmenge der pharmazeutischen Zusammensetzung oder das Verhältnis von Wassergehalt/Wirkstoff, gemessen in seiner wasserfreien Form, ist bis zu 31,5 % (w/w);
wobei der Wirkstoff das Distrontiumsalz der Säure 2-(N,N-Di(carboxymethyl)amino)-3-cyano-4-carboxymethyl-5-carboxythiophen ist und wobei die D50 der Granulate zur unmittelbaren Freisetzung von 140 bis 800 µm ist.

2. Pharmazeutische Zusammensetzung gemäß dem vorhergehenden Anspruch, wobei die pharmazeutische Zusammensetzung ungefähr 50 % (w/w) des Wirkstoffs, gemessen als wasserfreie Form, in Bezug auf die Gesamtmenge der pharmazeutischen Zusammensetzung umfasst; der Wirkstoff der einzige aktive Bestandteil in der pharmazeutischen Zusammensetzung in Form eines Tetrahydrats, Heptahydrats, Octahydrats, einer Mischung aus Tetrahydrat/Heptahydrat oder einer Mischung aus Octahydrat/Heptahydrat ist.

3. Die pharmazeutische Zusammensetzung gemäß einem der vorhergehenden Ansprüche, wobei der Wirkstoff das Distrontiumsalz der Säure 2-(N,N-Di(carboxymethyl)amino)-3-cyano-4-carboxymethyl-5-carboxythiophen ist und die pharmazeutische Zusammensetzung von 2,0 bis 15,0 % (w/w) Wassergehalt in Bezug auf die Gesamtmenge der pharmazeutischen Zusammensetzung umfasst, das Verhältnis Wassergehalt/Wirkstoff, gemessen als wasserfreie Form, von 3,6 bis 30,5 % (w/w) reicht, die pharmazeutische Zusammensetzung von 8,0 bis 13,0 % (w/w) Wassergehalt in Bezug auf die Gesamtmenge der pharmazeutischen Zusammensetzung umfasst, oder das Verhältnis Wassergehalt / Wirkstoff, gemessen als wasserfreie Form, von 15,0 bis 26,0 % (w/w) reicht.

4. Die pharmazeutische Zusammensetzung gemäß einem der vorhergehenden Ansprüche, wobei der Wirkstoff das Distrontiumsalz der Säure 2-(N,N-Di(carboxymethyl)amino)-3-cyano-4-carboxymethyl-5-carboxythiophen ist und der D50 der Granulate zur unmittelbaren Freisetzung von 140 bis 800 µm reicht und der D90 unterhalb von 1600 µm liegt, der D50 der Granulate zur unmittelbaren Freisetzung von 300 bis 500 µm reicht und der D90 unterhalb von 1000 µm liegt, oder der Carr Index des Granulats unterhalb von 25 liegt.

5. Die pharmazeutische Zusammensetzung gemäß einem der vorhergehenden Ansprüche, wobei der Wirkstoff das Distrontiumsalz der Säure 2-(N,N-Di(carboxymethyl)amino)-3-cyano-4-carboxymethyl-5-carboxythiophen ist und die Zusammensetzung umfasst: wenigstens ein Verdünnungsmittel und wenigstens ein Bindemittel; oder eine Gesamtmenge an Verdünnungsmittel oder Verdünnungsmitteln zwischen 15 und 35 % (w/w) und die Zusammensetzung eine Gesamtmenge an Bindemittel oder Bindemitteln zwischen 3 und 25 % (w/w) umfasst.

6. Die pharmazeutische Zusammensetzung gemäß einem der vorhergehenden Ansprüche, wobei der Wirkstoff das Distrontiumsalz der Säure 2-(N,N-Di(carboxymethyl)amino)-3-cyano-4-carboxymethyl-5-carboxythiophen ist und die Zusammensetzung umfasst: wenigstens ein Verdünnungsmittel, wenigstens ein Bindemittel und wenigstens ein Süßungsmittel; oder eine Gesamtmenge an Verdünnungsmittel oder Verdünnungsmitteln zwischen 15 und 35 % (w/w), eine Gesamtmenge an Bindemittel oder Bindemitteln zwischen 3 und 25 % (w/w) und eine Gesamtmenge an Süßungsmittel oder Süßungsmitteln zwischen 0,05 und 2 % (w/w).

7. Die pharmazeutische Zusammensetzung gemäß einem der vorhergehenden Ansprüche, wobei der Wirkstoff das Distrontiumsalz der Säure 2-(N,N-Di(carboxymethyl)amino)-3-cyano-4-carboxymethyl-5-carboxythiophen ist und die Menge an Wirkstoff, gemessen als wasserfreie Form, von 1,75 bis 2,25 g ist und/oder die pharmazeutischen Zusammensetzungen durch Nassgranulationstechniken hergestellt werden.

8. Die pharmazeutische Zusammensetzung gemäß einem der vorhergehenden Ansprüche, wobei die pharmazeutische Zusammensetzung in Form eines Stick-Packs vorliegt, wobei optional die pharmazeutische Zusammensetzung wenigstens die zwei folgenden Teile umfasst:
i) ein Granulat, enthaltend das Distrontiumsalz der Säure 2-(N,N-Di(carboxymethyl)amino)-3-cyano-4-carboxymethyl-5-carboxythiophen, bevorzugt umfasst das Granulat eine Menge des Distrontiumsalzes der Säure 2-(N,N-Di(carboxymethyl)amino)-3-cyano-4-carboxymethyl-5-carboxythiophen, gemessen als wasserfreie Form, die von 1,75 bis 2,25 g in jedem Stick-Pack reicht;
ii) eine Stick-Pack-Verpackung, wobei die Stick-Pack-Verpackung wenigstens eine versiegelte laminierte Aluminiumfolie enthält, wobei die Verpackung bevorzugt eine versiegelte laminierte Aluminiumfolie mit weniger als 20 µm Dicke enthält.

9. Eine Karton-/Pappkartonschachtel mit Patienten-Informationsmerkblättern in Packungsgrößen von wenigstens 4 Einheiten einer pharmazeutischen Zusammensetzung, bevorzugt in Stick-Packs abgepackt, gemäß einem der vorhergehenden Ansprüche zur pharmazeutischen Zusammensetzung.

10. Ein Verfahren zur Herstellung einer pharmazeutischen Zusammensetzung in Form von Granulaten zur unmittelbaren Freisetzung gemäß einem der vorhergehenden Ansprüche, wobei die pharmazeutische Zusammensetzung umfasst:
a) einen Wirkstoff von 25 bis 85 % (w/w) in Bezug auf die Gesamtmenge der pharmazeutischen Zusammensetzung;
b) bis zu 15,7 % (w/w) Wassergehalt in Bezug auf die Gesamtmenge der pharmazeutischen Zusammensetzung, oder das Verhältnis Wassergehalt/Wirkstoff, gemessen in wasserfreier Form, ist bis zu 31,5 % (w/w);
wobei der Wirkstoff das Distrontiumsalz der Säure 2-(N,N-Di(carboxymethyl)amino)-3-cyano-4-carboxymethyl-5-carboxythiophen ist und wobei der D50 der Granulate zur unmittelbaren Freisetzung von 140 bis 800 µm reicht;
wobei das Verfahren zur Herstellung der pharmazeutischen Zusammensetzung in Form von Granulaten zur unmittelbaren Freisetzung die Schritte umfasst:
i) Sieben der intragranulären Bestandteile, wenn dienlich, um bezüglich der Größe einheitliche Partikel intragranulärer Bestandteile zu bilden;
ii) Mischen der Partikel aus intragranulären Bestandteilen und des Distrontiumsalzes der Säure 2-(N,N-Di(carboxymethyl)amino)-3-cyano-4-carboxymethyl-5-carboxythiophen, bevorzugt in einem Granulierungsmischer; Granulieren der erhaltenen Mischung mit wenigstens einem Bindemittel, wobei das Bindemittel bevorzugt Wasser und einen Bindungswirkstoff enthält und weiter bevorzugt im Wesentlichen aus Wasser und Maltodextrin besteht;
iii) optional und/oder wenn erforderlich, Durchführen eines Siebens des Granulats, das in Schritt (ii) erhalten wird;
iv) Trocknen des Granulats aus den vorhergehenden Schritten (ii) oder (iii) bis zu einem Endpunkt von bis zu 15,7 % (w/w) Wassergehalt in Bezug auf die Gesamtmenge des Granulats, oder das Verhältnis Wassergehalt/Wirkstoff, gemessen in wasserfreier Form, ist bis zu 31,5 % (w/w), wobei das Trocknen bevorzugt in einem Fließbett bei einer Einlasstemperatur zwischen 35 und 105°C durchgeführt wird.

11. Das Verfahren gemäß einem der vorhergehenden Verfahrensansprüche, wobei in Schritt (iv) das Granulat aus dem vorhergehenden Schritt (ii) oder (iii) bis zu einem Endpunkt-Wassergehalt von 2,0 bis 15,0 % (w/w) in Bezug auf die Gesamtmenge der pharmazeutischen Zusammensetzung getrocknet wird, oder das Verhältnis Wassergehalt/Wirkstoff, gemessen in wasserfreier Form, von 3,6 bis 30,5 % (w/w) ist.

12. Das Verfahren gemäß einem der vorhergehenden Verfahrensansprüche, wobei der Wirkstoff, der in Schritt (ii) verwendet wird, oder das Distrontiumsalz der Säure 2-(N,N-Di(carboxymethyl)amino)-3-cyano-4-carboxymethyl-5-carboxythiophen, eine mittlere Teilchengröße von 10 bis 50 µm hat, wie durch Laserlichtdiffraktion gemessen.

13. Das Verfahren gemäß einem der vorhergehenden Verfahrensansprüche, wobei wenigstens 10.000 Einheiten pharmazeutische Zusammensetzung hergestellt werden und jede Einheit der pharmazeutischen Zusammensetzung eine Menge des Wirkstoffs des Distrontiumsalzes der Säure 2-(N,N-Di(carboxymethyl)amino)-3-cyano-4-carboxymethyl-5-carboxythiophen, gemessen in wasserfreier Form, von 1,75 bis 2,25 g enthält.

14. Eine pharmazeutische Charge von wenigstens 10.000 Einheiten der pharmazeutischen Zusammensetzung gemäß einem der vorhergehenden Produktansprüche, wobei die Menge des Wirkstoffs, gemessen in wasserfreier Form, von 1,75 bis 2,25 g in jeder Einheit reicht.

15. Die pharmazeutische Charge gemäß dem vorhergehenden Anspruch, wobei die Einheiten in Form von Stick-Packs vorliegen, wobei jede Einheit wenigstens die zwei folgenden Teile umfasst:
i) ein Granulat, enthaltend das Distrontiumsalz der Säure 2-(N,N-Di(carboxymethyl)amino)-3-cyano-4-carboxymethyl-5-carboxythiophen;
ii) eine Stick-Pack-Verpackung, wobei die Stick-Pack-Verpackung wenigstens eine versiegelte laminierte Aluminiumfolie umfasst, bevorzugt mit weniger als 20 µm Dicke.

## Revendications

1. Composition pharmaceutique sous la forme de granulés à libération immédiate, où la composition pharmaceutique comprend:
a) un agent de 25 à 85 % p/p par rapport à la quantité totale de la composition pharmaceutique;
b) jusqu'à 15,7 % p/p de teneur en eau par rapport à la quantité totale de la composition pharmaceutique ou le rapport teneur en eau/agent mesuré sous forme anhydre est de jusqu'à 31,5 % p/p;
dans laquelle l'agent est le sel de distrontique de l'acide 2-(N,N-di(carboxyméthyl)amino)-3-cyano-4-carboxyméthyl-5-carboxythiophène, et dans laquelle le D50 des granulés à libération immédiate se situe dans la plage de 140 à 800 microns.

2. Composition pharmaceutique selon la revendication précédente, dans laquelle la composition pharmaceutique comprend environ 50 % p/p de l'agent mesuré sous forme anhydre par rapport à la quantité totale de la composition pharmaceutique ; l'agent est le seul principe actif dans la composition pharmaceutique ou l'agent est sous forme de tétrahydrate, heptahydrate, octahydrate, mélange de tétrahydrate/heptahydrate ou mélange d'octahydrate/ heptahydrate.

3. Composition pharmaceutique selon l'une quelconque des revendications précédentes, dans laquelle l'agent est le sel distrontique de l'acide 2-(N,N-di(carboxyméthyl)amino)-3-cyano-4-carboxyméthyl-5-carboxythiophène et la composition pharmaceutique comprend de 2,0 à 15,0 % p/p de teneur en eau par rapport à la quantité totale de la composition pharmaceutique, le rapport teneur en eau/agent mesuré sous forme anhydre se situe dans la plage de 3,6 à 30,5 % p/p, la composition pharmaceutique comprend de 8,0 à 13,0 % p/p de teneur en eau par rapport à la quantité totale de la composition pharmaceutique ou le rapport teneur en eau/agent mesuré sous forme anhydre se situe dans la plage de 15,0 à 26,0 % p/p.

4. Composition pharmaceutique selon l'une quelconque des revendications précédentes, dans laquelle l'agent est le sel distrontique de l'acide 2-(N,N-di(carboxyméthyl)amino)-3-cyano-4-carboxyméthyl-5-carboxythiophène et le D50 des granulés à libération immédiate se situe dans la plage de 140 à 800 microns et le D90 est inférieur à 1600 microns, le D50 des granulés à libération immédiate se situent dans la plage de 300 à 500 microns et le D90 est inférieur à 1000 microns ou l'index de Carr du granulat est inférieur à 25.

5. Composition pharmaceutique selon l'une quelconque des revendications précédentes, dans laquelle l'agent est le sel distrontique de l'acide 2-(N,N-di(carboxyméthyl)amino)-3-cyano-4-carboxyméthyl-5-carboxythiophène et la composition comprend : au moins un diluant et au moins un liant ; ou une quantité totale d'un diluant ou de diluants entre 15 et 35 % p/p et la composition comprend une quantité totale d'un liant ou de liants entre 3 et 25 % p/p.

6. Composition pharmaceutique selon l'une quelconque des revendications précédentes, dans laquelle l'agent est le sel distrontique de l'acide 2-(N,N-di(carboxyméthyl)amino)-3-cyano-4-carboxyméthyl-5-carboxythiophène et la composition comprend : au moins un diluant, au moins un liant et au moins un agent édulcorant ; ou une quantité totale d'un diluant ou de diluants entre 15 et 35 % p/p, une quantité totale d'un liant ou de liants entre 3 et 25 % p/p et une quantité totale d'un agent édulcorant ou d'agents édulcorants entre 0,05 et 2 % p/p.

7. Composition pharmaceutique selon l'une quelconque des revendications précédentes, dans laquelle l'agent est le sel distrontique de l'acide 2-(N,N-di(carboxyméthyl)amino)-3-cyano-4-carboxyméthyl-5-carboxythiophène et la quantité d'agent mesuré sous forme anhydre est de 1,75 à 2,25 grammes et/ou les compositions pharmaceutiques sont fabriquées par des techniques de granulation humide.

8. Composition pharmaceutique selon l'une quelconque des revendications précédentes, dans laquelle la composition pharmaceutique est sous la forme de stick-pack, éventuellement la composition pharmaceutique comprend au moins les deux parties suivantes:
i) un granulat comprenant le sel distrontique de l'acide 2-(N,N-di(carboxyméthyl)amino)-3-cyano-4-carboxyméthyl-5-carboxythiophène, de préférence le granulat comprend une quantité du sel distrontique de l'acide 2-(N,N-di(carboxyméthyl)amino)-3-cyano-4-carboxyméthyl-5-carboxythiophène mesuré sous forme anhydre située dans la plage de 1,75 à 2,25 grammes dans chaque stick-pack;
ii) un emballage de type stick-pack, où l'emballage comprend au moins une feuille stratifiée d'aluminium scellée, où de préférence l'emballage comprend une feuille stratifiée d'aluminium scellée de moins de 20 microns d'épaisseur.

9. Boîte en carton avec des brochures d'information pour le patient d'au moins 4 unités de composition pharmaceutique, de préférence conditionnées en stick-packs, selon l'une quelconque des revendications précédentes de composition pharmaceutique.

10. Procédé de fabrication d'une composition pharmaceutique sous la forme de granulés à libération immédiate selon l'une quelconque des revendications précédentes, dans laquelle la composition pharmaceutique comprend:
a) un agent de 25 à 85 % p/p par rapport à la quantité totale de la composition pharmaceutique;
b) jusqu'à 15,7 % p/p de teneur en eau par rapport à la quantité totale de la composition pharmaceutique ou le rapport teneur en eau/agent mesuré sous forme anhydre est de jusqu'à 31,5 % p/p;
dans laquelle l'agent est le sel distrontique de l'acide 2-(N,N-di(carboxyméthyl)amino)-3-cyano-4-carboxyméthyl-5-carboxythiophène, et dans laquelle le D50 des granulés à libération immédiate se situe dans la plage de 140 à 800 microns;
dans laquelle le procédé de fabrication de la composition pharmaceutique sous la forme de granulés à libération immédiate comprend les étapes suivantes:
i) le tamisage, lorsque c'est approprié, des composants intragranulaires pour former des particules de taille uniforme des composants intragranulaires;
ii) le mélange des particules des composants intragranulaires et du sel distrontique de l'acide 2-(N,N-di(carboxyméthyl)amino)-3-cyano-4-carboxyméthyl-5-carboxythiophène, de préférence dans un granulateur-mélangeur ; la granulation du mélange obtenu avec au moins un liant, de préférence le liant comprend de l'eau et un agent liant, de manière davantage préférée il est constitué essentiellement d'eau et de maltodextrine;
iii) éventuellement et/ou si nécessaire, la réalisation d'un tamisage du granulat obtenu dans l'étape (ii);
iv) le séchage du granulat de l'étape précédente (ii) ou (iii) jusqu'à un critère de jusqu'à 15,7 % p/p de teneur en eau par rapport à la quantité totale du granulat ou le rapport teneur en eau/agent mesuré sous forme anhydre est de jusqu'à 31,5 % p/p, dans laquelle le séchage est réalisé de préférence dans un lit fluide à une température d'entrée entre 35 et 105 °C.

11. Procédé selon les revendications précédentes de procédé, dans lequel dans l'étape (iv), le granulat de l'étape précédente (ii) ou (iii) est séché jusqu'à un critère de teneur en eau de 2,0 à 15,0 % p/p par rapport à la quantité totale de la composition pharmaceutique ou le rapport teneur en eau/agent mesuré sous forme anhydre est de 3,6 à 30,5 % p/p.

12. Procédé selon l'une quelconque des revendications précédentes de procédé, dans lequel l'agent utilisé dans l'étape (ii) ou le sel distrontique de l'acide 2-(N,N-di(carboxyméthyl)amino)-3-cyano-4-carboxyméthyl-5-carboxythiophène a une taille moyenne de particule de 10 à 50 microns mesurée par diffraction de lumière laser.

13. Procédé selon l'une quelconque des revendications précédentes de procédé, dans lequel au moins 10 000 unités de composition pharmaceutique sont fabriquées et chaque unité de composition pharmaceutique comprend une quantité d'agent, le sel distrontique de l'acide 2-(N,N-di(carboxyméthyl)amino)-3-cyano-4-carboxyméthyl- 5-carboxythiophène, mesuré sous forme anhydre de 1,75 à 2,25 grammes.

14. Lot pharmaceutique d'au moins 10 000 unités de la composition pharmaceutique selon l'une quelconque des revendications précédentes de produit et la quantité d'agent mesuré sous forme anhydre se situe dans la plage de 1,75 à 2,25 grammes dans chaque unité.

15. Lot pharmaceutique selon la revendication précédente, dans laquelle les unités sont sous la forme de stick-pack, dans laquelle chaque unité comprend au moins les deux parties suivantes :
i) un granulat comprenant le sel distrontique de l'acide 2-(N,N-di(carboxyméthyl)amino)-3-cyano-4-carboxyméthyl-5-carboxythiophène;
ii) un emballage de type stick-pack, où l'emballage comprend au moins une feuille stratifiée d'aluminium scellée, de préférence de moins de 20 microns d'épaisseur.
